Europäisches Patentamt

European Patent Office

Office européen des brevets

(11) Publication number: **0 267 625**
**A2**

# EUROPEAN PATENT APPLICATION

(21) Application number: **87116801.9**

(22) Date of filing: **13.11.87**

(51) Int. Cl.⁴: **G01N 33/543** , //G01N33/569

(30) Priority: **14.11.86 US 930428**

(43) Date of publication of application:
**18.05.88 Bulletin 88/20**

(84) Designated Contracting States:
**DE FR GB IT**

(71) Applicant: **Levine, Robert Aaron**
**31 Pilgrim Lane**
**Guilford, Connecticut 06437(US)**

Applicant: **Wardlaw, Stephen Clark**
**191 North Cove Road**
**Old Saybrook Connecticut 06475(US)**

(72) Inventor: **Levine, Robert Aaron**
**31 Pilgrim Lane**
**Guilford, Connecticut 06437(US)**
Inventor: **Wardlaw, Stephen Clark**
**191 North Cove Road**
**Old Saybrook Connecticut 06475(US)**

(74) Representative: **Klunker . Schmitt-Nilson .**
**Hirsch**
**Winzererstrasse 106**
**D-8000 München 40(DE)**

(54) **Method of assaying biological fluid samples.**

(57) A solid surface as the inside of a receptacle or a paper dipstick is coated with a particular adherent material, such as an antibody, enzyme, antigen, or the like. A biological fluid sample, such as blood, is then brought into contact with the coated surface, and a target constituent of the sample, such as reticulocyte cells, is adhered to the coated surface, due to the presence of a specific binding characteristic possessed by the target constituent for which the adherent material has an affinity. The non-adhered portion of the sample is then removed. Intraparticulate material, such as hemoglobin, in the case of reticulocytes, is released from the adhered constituent and assayed.

EP 0 267 625 A2

0 267 625

## "METHOD OF ASSAYING BIOLOGICAL FLUID SAMPLES"

This invention relates broadly to the obtaining of information about a target membranal particulate constituent sub-population having a binding characteristic, and which is contained in a biological fluid mixture. More particularly, a preferred embodiment of this invention concerns a relatively simple and reliable method for detecting and/or quantitating reticulocytes in vertebrate blood.

The ability of a physician to rapidly diagnose and then administer to various blood-related diseases or abnormalities depends in large part on how quickly and accurately he or she can detect and/or enumerate certain sub-populations in the blood or other biologic fluid of the patient. For example, the diagnosis of malaria requires the selection and detection and/or enumeration of a sub-population of a relatively homogeneous group of cells, namely, the malaria-infected erythrocytes. Likewise, diagnosing the cause of anemia, i.e., whether it is due to excessive bleeding or to inadequate blood production, requires an accurate determination of the reticulocyte count in the blood. If an anemic patient has a hematocrit of 30%, a high reticulocyte count would signify hemolysis, or excessive blood loss; whereas, bone marrow suppression would be indicated by a low reticulocyte count.

With about 0.85% of the erythrocytes (RBC) normally being lost and replaced with new cells every day, the best measure of new RBC production is the reticulocyte count. Unfortunately, at present the determination of reticulocyte count may be prone to substantial error or variation. To illustrate, the normal values quoted in the literature for a reticulocyte count range from 0.5% to 1.5% or a variation of 300%. This substantial variation is due, not to a three-fold difference in RBC production in normal subjects, but rather to error in measurement.

The error in determining the reticulocyte count is perhaps attributable in part to the fact that the reticulocytes, which may be assumed, for purposes of understanding their relationship to RBC production, to be RBC's that are one day old, loose their intracellular reticular pattern gradually. As a consequence, two-day old erythrocytes may be considered to be reticulocytes by one observer but not by another. Nevertheless, when a physician is given a reticulocyte count analysis, he or she may well assume that it is an accurate count, when in fact it may not be, depending on the particular observer's view of what constitutes a reticulocyte and what does not. It can thus be appreciated that a variation in observed reticulocyte count may not necessarily signify a corresponding variation in RBC production.

It is, therefore, readily apparent that if an accurate, easy-to-perform reticulocyte count were available, it would be a great advance in diagnostic hematology.

Various methods have been disclosed in the prior art for the detection or determination of microorganisms or cell sub-populations in liquid media. One approach involves the use of specialized, sophisticated equipment. See for example U.S. Patent No. 4,599,307 to Saunders et al. In general, due to the costly equipment required, not to mention the rather complex procedure, such methods have not been accepted and used on any measurable scale in the detection/enumeration of cell sub-populations in vertebrate blood.

Another approach, on which a large number of patented methods have been based, makes use of antibodies or antigens having a specific affinity for a particular cell sub-population in a blood sample. Typically, the sample is brought into intimate contact with the antibodies after the latter have been placed in a tube or coated onto a surface. After the cells have become adhered to the antibodies, the remainder of the sample is removed and the adherent cells are identified by available methods. Illustrative references which utilize this approach are U.S. Patent 4,070,243 to Teodorescu et al, U.S. Patent 4,407,943 to Cole et al, and U.S. Patent 4,591,570 to Chang. However, the methods disclosed in these patents are generally aimed at detecting the presence of certain cell sub-populations and they fall short of providing a reliable method for quantitating a particular cell sub-population in a sample.

Utilizing a quantitative approach, U.S. Patent 4,592,994 to Mattlasson discloses a method for detecting and quantitating microorganisms and unicellular organisms in a sample. After isolating the organisms in question by means of selectively adherant antibodies, Mattlasson teaches the concept of adding a nutrient in which the adherant organisms can metabolize. After an appropriate incubation period, e.g., 30-120 minutes, the nutrient medium is analyzed by spectrophotometry. Based on the value read in that analysis, it is then possible, according to Mattlasson, to determine the number of adhered organisms by reference to an appropriate calibration curve.

The patent to Mattlasson is noteworthy in that it provides a quantitative method for measuring certain live organisms in a sample. However, in addition to being time-consuming and requiring precise control of certain parameters which may affect the metabolism step, the Mattlasson method has no applicability to the detection/quantitation of cell sub-populations in blood.

U.S. Patent No. 4,027,660 granted June 7, 1977 to Levine et al discloses a method for measuring

2

reticulocytes in a centrifuged sample of blood contained in a capillary tube.

In view of the foregoing, there is still a need for another simple yet accurate method for the detection and/or enumeration of cell sub-populations in media containing other not readily distinguishable cells ,such as blood.

We have invented a method which fulfills this need. In accordance with this invention, cell sub-populations in a sample can be simply and accurately detected and quantitated by a method which comprises:

(a) isolating the cell sub-population in question from the sample with a specific binder;

(b) releasing a detectable or measurable constituent of the isolated cells; and

(c) detecting, or measuring, the concentration of the constituent

More specifically, isolation of the cell sub-population which is to be detected or quantitated can be achieved by a material which binds the sub-population, as for example, an enzyme or an antibody. The preferred approach involves the use of antibodies, antigens, or other materials which have specific affinity for, and will adhere selectively to, the cells in question. For simplicity and quick analysis, it is also preferable to use a solid or semi-solid surface of a small container, as a receptacle to bring the sample into contact with the selectively adherent antibodies, antigens or the like. For example the solid or semi-solid surface may be made of solid glass, glass beads, paper, plastic. or an appropriate membrane material or the like; and the small container conveniently can be a small test tube preferably made of glass or an appropriate plastic material. The use of a test tube or the like is one preferred manner of practicing the method of this invention. Another preferred means for carrying the antibody or the like is a paper dip stick which is coated with the antibody.

In practice, a coating comprised of the selectively adherent material, be it antibodies, antigens or the like, is applied to the surface of the receptacle or to a dipstick. In the case of a tubular receptacle, the coating is applied to the inner surface of the tube. A suitable method for coating the interior of the receptacle, or other surface, is disclosed in U.S. Patent 4,066,512 to Lai et al, the disclosure of which is incorporated herein by reference.

In accordance with this invention, in order to accurately quantitate the target cells in question, it is preferable that the coating contain a sufficient amount of the selectively adherent material such as to bind or capture a relatively constant proportion of the target cells. For practical reasons, therefore, it may be preferable or advisable to use an amount of the binder which is in excess of the needed amount. The sample is brought into contact with the coating to bring about the adhesion of the needed proportion of the cells in question to the selectively adherent material. Ordinarily this can be achieved by maintaining intimate contact between the sample and the coating for a few minutes with gentle agitation. Thereafter, the remaining sample, containing unadhered material, is removed or separated from the coating such as by decanting and gentle washing with an isotonic diluent or the like.

The adhered cells are then treated to dislodge or release their constituents, the latter being captured for subsequent assay. For example, the cells may be ruptured or lysed using conventional methods, and the released constituents collected. Thereafter, one or more of these constituents are measured to determine the number or even merely the presence of adherent cells. Where it is merely desired to determine the presence or absence of the particular cell sub-population, any quantity of the particular constituent would be a positive test; whereas, if it is desired to quantitate the cell sub-population, this can be achieved by measuring total constituent released and, assuming a uniform distribution of the constituent, comparing the measured amount with the amount of total constituent in the entire cell population. The released constituent could be hemoglobin, potassium, DNA, RNA, enzymes, or some other intercellular component.

Thus in accordance with the preferred embodiments of the invention, the method for determining and quantitating the reticulocyte cell sub-population in a blood sample comprises the following steps:

(a) applying to a solid surface a coating containing an antireticulocyte antibody, which is an anti transferrin surface receptor antibody, and which has specific affinity for, and, as regards the red cells, will adhere only to, the reticulocyte cells, in an amount sufficient to bind all, or a predetermined detectable quantity, of the reticulocyte sub-population contained in said sample,

(b) bringing the sample into contact with the coating such as to cause the predetermined amount of the reticulocyte cells to adhere to the coating;

(c) removing or separating the remaining non-adherant portions of the sample from the adhered cells;

(d) lysing the adhered cells to release their hemoglobin constituent; and

(e) enumerating or measuring the concentration of the released hemoglobin in a hemoglobinometer and, on the basis of such measurement or enumeration, determining the total count of the reticulocyte sub-population contained in said sample.

The hemoglobin content is assayed by the conventional cyanmethemoglobin determination at 560 nm, or by direct spectrophometry in the Soret band (415 nm). For maximum sensitivity, the pseudo-peroxidase activity of the hemoglobin can be measured using tetramethylbenzidine or some other color developing agent.

The number of reticulocytes in a blood sample can be determined using the method of this invention in the following illustrative series of steps. Antibodies having a specific affinity for the reticulocytes are coated onto the inner surface of a test tube. A diluted 30 ul sample of whole blood is added to the tube which is then gently agitated to allow the red cells to come into intimate contact with the antibodies and to allow the reticulocytes therein to adhere to the antibody coating. After a few minutes, the tube is decanted and gently washed with an isotonic diluent to remove all non-adhered erythrocytes. A lysing agent such as saponin is then added to release the hemoglobin from the adhered erythrocytes, whereupon the released hemoglobin is then measured in a spectrophometer.

The following calculations illustrate the sensitivity of the method of this invention:

| Mean RBC diameter | 7.2 u |
|---|---|
| RBC attachment density (calc) | $2.0 \times 10$ 6th power |
| Surface area of 1x2 cm tube | 7.0 cm squared |
| Capturable RBC's on tube | $1.4 \times 10$ 7th power |
| Standard hemoglobin absorbance: | |
| CyanmeHgb-560nm (Ref) | $.019 \times 10$ 6th power RBC/ml |
| Soret band-415nm(Ref) | $.259 \times 10$ 6th power RBC/ml |

The following illustrative calculations assume 30ul of whole blood added to the tube, with a final volume of 1.0ml:

RBC content in 30ul sample      $1.35 \times 10$ eighth power

Normal reticulocyte level (1.0%)      $1.35 \times 10$ sixth power (RBC)

CyanmetHgb absorbence of 1.0% sample      .026

"Measured" Hgb Conc.      1.03 g/dl

Maximum reticulocyte level (10.0%)      $.35 \times 10$ seventh power (RBC)

(Note: "Measured" hemoglobin = "reticulocyte" count)

The above illustration demonstrates that the method of this invention is capable of measuring reticulocytes in a standard hemoglobinometer with a sensitivity limit of 0.1% and a maximum reticulocyte percentage of approximately 10. This is the sensitivity and dynamic range required for clinical work. However, if necessary, a 14 fold boost in sensitivity can be achieved by using the Soret band absorbance.

It is readily apparent from the aforesaid description of this invention that the broad concept and method disclosed herein is not limited to the detection/enumeration of sub-populations of blood cells. Rather, this invention has utility in a variety of other applications. For example, this invention can be used in the detection of parasites or other immunologically distinct particulates or cells in complex mixtures, such as urine, spinal fluid, or the like. Additionally, this invention could be used to detect certain other blood conditions, as, for example in an assay of glycosylated hemoglobin in a blood sample in order to determine severity of diabetes.

Since many changes and variations of the disclosed embodiments of the invention may be made without departing from the inventive concept, it is not intended to limit the invention otherwise than as required by the appended claims.

**Claims**

1. A method of detecting and quantitating a sub-population of biologic particles in a sample containing morphologically similar biologic particles which comprises:

(a) applying to a surface a coating containing an amount of antibodies, antigens or other material which has an affinity for the particles in said sub-population, said amount being sufficient to bind a relativly constant detectable fraction of said sub-population of particles contained in said sample to said surface;

(b) bringing said sample into contact with said coating so as to cause said fraction of said particle sub-population to adhere to said coating;

(c) removing or separating the remaining non-adherent portions of said sample from said surface;

(d) dislodging a measurable constituent from the adhered particles; and

(e) enumerating or measuring the concentration of said constituent.

2. The method of Claim 1 wherein said surface is inside of a receptacle.

3. The method of Claim 1 or 2 wherein said amount is equal to or in excess of that required to bind all of the particles in said sub-population.

4. The method of any of Claims 1 to 3 wherein step (d) is carried our by rupturing or lysing said adhered particles, thereby releasing the measurable constituent.

5. The method of any of Claims 2 to 4 wherein said remaining non-adherent portion of said sample is removed by washing said inside surface of said receptacle with a diluent.

6. The method of Claim 5 wherein step (c) is carried out by rupturing or lysing said adhered particles, thereby releasing said measurable constituent.

7. The method of Claim 6 wherein said amount is in excess of the amount required to bind all of the particles in said sub-population.